# EUROPEAN PATENT APPLICATION

(11) **EP 1 045 024 A1**
(43) Date of publication of application: **18.10.2000**
(21) Application number: 99201134.6
(22) Date of filing: 12.04.1999
(51) Int. Cl.: C12N 5/06, C12M 3/06, A61K 35/407, A61L 27/00

(54) **Use of human hepatocyte cell line for the treatment of chronic or acute liver failure**

(71) Applicant: K.U. Leuven Research & Development, 3000 Leuven (BE)
(72) Inventor: The designation of the inventor has not yet been filed
(74) Representative: Van Someren, Petronella F. H. M.

(57) **Abstract**

The present invention relates to a cell line of human immortalized hepatocytes for medical use. In particular, the invention relates to a cell line of human immortalized hepatocytes for the treatment of liver failure. More specifically, the invention relates to a cell line of human hepatocytes for use in a bioartificial liver system for the treatment of chronic or acute liver failure. The invention further relates to the use of said cell line for the preparation of a bioartificial liver system for the treatment of liver failure.

Furthermore, the invention relates to a bioartificial liver system provided with said cell line, and the use thereof for the treatment liver failure.

According to the present invention it has for the first time been demonstrated that a cell line of human immortalized hepatocytes can effectively be used for the treatment of liver failure, resulting in a prolongation of survival accompanied by an improvement of biochemical parameters and neurological status.

## Description

The present invention relates to a cell line of human hepatocytes for medical use. In particular, the invention relates to a cell line of human hepatocytes for the treatment of liver failure. More specifically, the present invention relates to a cell line of human hepatocytes for use in a bioartificial liver system for the treatment of liver failure. The invention further relates to the use of a cell line of human hepatocytes for the preparation of a bioartificial liver system for the treatment of liver failure, as well as to a bioartificial liver system provided with a cell line of human immortalized hepatocytes, and its use for the treatment of liver failure.

Normally, after damage of the liver tissue the liver is capable of rapid and complete regeneration. However, when the regenerative process is compromised and the residual functional capacity of the diseased liver is not capable to sustain life, chronic or acute liver failure occurs.

The mortality of acute liver failure is very high. With intensive medical care mortality is still up to 80%. Orthotopic liver transplantation (OLTx) is currently the treatment of choice, by which a 1-year-survival of 90% can be achieved. There are, however, several major problems associated with OLTx. First, OLTx can not be widely used because of the scarcity of donor livers. Second, the OLTx procedure is invasive and survivors face a lifetime use of immunosuppressive agents and medical supervision. Moreover, costs involved with OLTx are very high.

OLTx thus is a drastic and expensive solution for an acute, potentially self-limiting disease. This therapeutic option creates a dilemma: transplantation performed early in the course of ALF has the best outcome, but some patients undergo needless surgery because early transplantation neglects the possibiliby of regeneration. Moreover, due to the chronic scarcity of donor organs some patients die while on the waiting list for OLTx.

The above mentioned problems highlight the need for some form of liver assist in chronic or acute liver failure. Many attempts have thus been made to develop an artificial liver (McGuire et al., Dig Dis 13, 379-388, 1995). However, as liver function is very complex and many of the functions still remain unclear, these attempts have failed.

More recently, so-called bioartificial livers (BALs) have been developed. BALs are extracorporeal devices which involve a biological (hepatocytes) and synthetic component (plastic housing shell). A bioartificial liver can be used as a liver assist in conditions of liver failure to span the time period needed for a donor liver to become available for the patient. In addition, when the liver is able to regenerate in this time period, transplantation might not be necessary.

Various cell types have been used in the development of a bioartificial liver. Although primary hepatocytes appear to maintain their phenotype under appropriate conditions, problems of scale and rapid loss of differentiated functions in culture are the major drawbacks. In addition, it is obvious that primary human hepatocytes can not be used for this purpose, since the donor organs available for cell isolation are very limited. Moreover, the maximal number of hepatocytes that can be isolated from a liver using the current available techniques is at best 5-10 billion cells. These donor organs could therefore better be used for liver transplantation.

Primary porcine hepatocytes have also been used in bioartificial liver systems (Flendrig et al., J Hepatol 26, 1379-1392, 1997; Rozga et al., Biotechnology and Bioengineering 43, 645-653, 1994). The use of non-human cells is, however, associated with several problems, including immunogenicity of animal proteins and transmission of potential hazardous elements such as porcine endogenous retroviruses.

Immortalized human cell lines thus are an attractive alternative to primary human hepatocytes. In this regard, human hepatoma or hepatoblastoma cell lines have been used. However, these cells have a decreased metabolic capacity limiting their use in bioartificial livers. In addition, as these cells are highly tumorigenic there is an obvious risk for the development of tumors in the patients when cells may escape from the bioartificial liver.

It is the general object of the present invention to provide a human hepatocyte cell line for medical use. A more specific object of the present invention is to provide a human hepatocyte cell line for use in a bioartificial liver for the treatment of liver failure, which cells do not lead to the aforementioned problems.

In the research that led to the present invention it has now been found that cell lines of human immortalized hepatoctes can be established. Normally, primary hepatocytes do not proliferate in vitro. Nevertheless, it was surprisingly found that albumin secreting colonies develop in long-term cultures. From such colonies several cell lines were investigated for hepatocyte functions, such as the ability to synthesize albumin and fibrinogen, bilirubin conjugation, cytochrome P₄₅₀ enzyme activity, presence of ALT, AST, γGT, LDH, and cytokeratin expression. When compared to hepatoblastoma cells, these cell lines were superior in several or almost all of the aforementioned characteristics. In addition, cellular activities, such as for example albumin and fibrinogen secretion, were comparable to primary human hepatocytes. The cells were also tested for tumorigenicity in SCID mice and proved to be non-tumorigenic.

It has already been demonstrated that cell lines of human hepatocytes can be developed and that these cells can be grown in large quantities in a hollow fibre system, which no longer is a monolayer culture (Fourneau et al., in: Bioartificial liver support systems: the critical issues, Crepaldi, Demetriou and Muraca, Eds; International up to date 208, CIC Edizioni Internationali, Rome, 1997). The actual use of these cell lines for medical purpose in general, and in particular for the preparation of a bioartificial liver for the effective treatment of liver failure is, however, not demonstrated in this article.

Hepatocytes of the cell line of the invention were used for the preparation of a bioartificial liver system for the treatment of liver failure and demonstrated to significantly increase survival in organisms suffering from liver failure. Therefore, the cell were seeded in a bioreactor as developed by Flendrig et al.(supra) and kept in culture until the total amount of hepatocytes was estimated to be about 5 billion. This bioartificial liver support system consists of a blood circuit with continuous plasma separation and a bioreactor circuit, and comprises a spirally wound nonwoven polyester 3D matrix for high density hepatocyte culture in small aggregates, and polypropylene oxygenation tubing for oxygen supply and CO₂ removal. The bioreactor is perfused with plasma, such that there is a direct contact between the plasma and the hepatocytes with low diffusion gradients, thereby more closely resembling the in vivo situation. Because of the plasmapheresis immune competent cells of the patient do not come in contact with the hepatocytes.

The bioartificial liver provided with a human hepatocyte cell line of the invention was tested in a experimental model of acute liver failure (ALF). This experimental model is based on total but reversible ischemia of the liver, which is a modification of the model of graded ischemia as described by de Groot at al. (J Surg Res 42, 92-100, 1987).

In short, pigs were subjected to xyphopubic laparotomy. The common bile duct and the common hepatic artery, together with the inbranching gastroduodenal artery, were isolated and a vessel loop was put around the hepatic artery just at the bifurcation into the right and left liver branch and around the common bile duct. In addition, the right gastric artery was surrounded by a vessel loop to prevent backflow. In some animals also the gastroduodenal artery was surrounded. These vessel loops were not tightened and were exteriorized through the abdominal wall. A side-to-side portocaval shunt was performed, followed by ligation of the portal vein close to the liver hilum to create a functional end-to-side shunt. After recovery, 72 hours postoperatively, the vessel loops around the common bile duct, hepatic artery, right gastric artery and, in case, around the gastroduodenal artery were tightened without anesthesia or sedation, causing a total liver ischemia. After 10 hours the loops were cut and pulled off.

Three groups of animals were tested. In group I pigs were not connected to any extracorporeal system. In group II the pigs were connected to a complete BAL system without hepatocytes. Group III pigs were treated with a BAL system, loaded with on average 5 billion cells of a humane hepatocyte cell line of the invention. These therapies were initiated 9 hours after induction of ischemia. Plasmapheresis was performed for maximally 72 hours or shorter if death occurred earlier.

All animals of group I died between 12 and 17 hours after the induction of ischemia. In all pigs death was due to a hepatic coma (ALF). Animals of group II died between 12 and 24 hours after the induction of ischemia. In contrast, with 24 hours of BAL treatment survival time of the pigs was increased up to 33 hours. When BAL treatment with a single bioreactor was continued until death, survival time reached 46 hours. When the bioreactor was renewed after 12 hours and the treatment continued for another 60 hours, the pigs survived with complete normalisation of behaviour and biochemical parameters. After sacrificing these pigs on day 7, histology the liver showed typical changes of regeneration. It has thus been demonstrated for the first time that survival of organisms suffering from severe liver failure can significantly be prolonged by the use of a cell line of human immortalized hepatocytes in a bioartificial liver.

Thus, according to the present invention a cell line of human immortalized hepatocytes is provided for medical use. In particular, according to the present invention a cell line of human immortalized hepatocytes is provided for the treatment of liver failure, more specifically, for use in a bioartificial liver for the treatment of liver failure. Treatment of subjects suffering form severe liver failure with a bioartificial liver loaded with human hepatocytes of the cell line of the invention, results in a significant prolongation of survival time which is accompanied by normalisation of biochemical parameters and an important improvement of neurological status.

The present invention further provides the use of a cell line of human hepatocytes for the preparation of a bioartificial liver system for the treatment of chronic or acute liver failure.

The human hepatocytes of the cell line of the invention are preferably non-tumorigenic in order to reduce the risk at the development of tumors in the patients when cells escape from the bioreactor and thus are introduced in the patient's body.

According to the present invention, differentiated hepatocyte functions, such as, for example, albumin and fibrinogen secretion, cytokeratin expression, and bilirubin conjugation are preferably preserved in the immortalized hepatocytes of the cell line at least to a level approximately comparable to that of primary hepatocytes. The levels of these functions in cell lines of the invention are shown in the Examples.

An important function, which hepatocytes rapidly loose after isolation is cytochrome P₄₅₀ enzyme activity. According to the present invention, advantageously cytochrome P₄₅₀ enzyme activity of the hepatocytes of the cell line of the invention is at least 30%, preferably at least 50% of the level of primary human hepatocytes.

Cell lines of human immortalized hepatocytes according to the invention are preferably obtained by a process as described in Example 1.

In a preferred embodiment of the present invention the cell line is the HHY-B cell line deposited on March 23, 1999 at the Belgian Coordinated Collections of Microorganisms (BCCM™) and having the deposit accesion no LMBP 4981 CB.

Advantageously, the cell line of human immortalized hepatocytes is used in a bioartificial liver system for the treatment of liver failure, which bioartificial liver system comprises a solid support for use in cell cultivation in vitro, comprising a 3D matrix and hollow fibres being permeable to at least gaseous oxygen and/or gaseous carbon dioxide.

The invention further provides a bioartificial liver system which is provided with a cell line of human immortalized hepatocytes of the invention, as well as its use for the treatment of liver failure. This treatment preferably consists of the extracorporal superfusion of human hepatocytes of the cell line, which are provided in a bioartificial liver system, by the body fluid of a patient suffering from liver failure, and subsequently returning the body fluid to the patient.

The invention also provides a permanent cell line of human hepatocytes for use in replacing and/or supporting in vivo liver function for increasing survival of patients with a liver disorder, by contacting a body fluid from the patient with the cells and returning the body fluid to the patient, wherein the cells are immortalized without viral transfection or transformation and are non-tumorigenic.

A cell line of human immortalized hepatocytes of the invention is preferably used for the treatment of liver failure in humans. However, it should be understood that according to the invention a cell line of human immortalized hepatocytes can be used for the treatment of animal sujects in general, in particular mammalian subjects, and more specifically human subjects. It is, however, preferred that for the treatment of a particular species, cell lines of that particular species are used.

According to the present invention a cell line is defined by a population cells of animal origin capable of dividing indefinitely in culture. The terms "permanent cell line" and "immortalized cell line" are used interchangeably throughout this specification.

The invention is further described in the following examples which are intended to illustrate the present invention, without limiting the scope of the invention.

### EXAMPLES

### EXAMPLE 1

### Isolation of human hepatocytes and selection of clones of immortalized cells.

### 1. Materials and Methods

### Isolation of human hepatocytes:

Human hepatocytes were isolated from human livers which were for some reason not suitable for transplantation. The liver tissue was obtained from postmortem donors with the approval of the Medical Ethical Committee for Human Experimentations of the Catholic University of Leuven. Human hepatocytes were isolated using a two-step collagenase perfusion method as described previously (Moshage et al., Biochem Biophys Res Comm 115, 112-117, 1988; Rijntjes et al., J Hepatol 3, 7-18, 1986).

All procedures were performed under sterile conditions. A part of the left lob of the human liver was used to isolate the hepatocytes. The large branches of the portal vein were cannulated with PVC tubings. Approximately 2 l of physiological salt solution (0.9% NaCl) at 4°C was perfused through the liver (rate 120-200 ml/min) to remove the remaining cold-storage solution which was used for sterile preservation and transportation of the tissue. This was followed by 3-4 l Ca²⁺/Mg²⁺-free buffer pH 7.4 (10 mM Hepes (4-[2-hydroxyethyl]-1-piperazine ethanesulfonic acid) (Sigma, St Louis, USA), 142 mM NaCl and 7 mM KCl) which was perfused through the liver at a rate of 120-200 ml/min at 37°C. As soon as the perfusion was started, it was observed that the tissue began to blanch as an indication that the blood in the tissue was replaced with the buffer. Subsequently, the tissue was perfused with 100 ml of a 100 mM Hepes buffer pH 7.6 containing 67 mM NaCl, 7 mM KCl, 20 mM CaCl₂ and 0.1% Collagenase B (Boehringer, Mannheim, Germany). Next, the liver tissue was perfused with 250 ml of a 100 mM Hepes buffer pH 7.6 containing 67 mM NaCl, 7 mM KCl, 10 mM CaCl₂ and 0.05% Collagenase B in a recirculation system for 20-30 minutes. All buffers, unless otherwise indicated, were maintained at 37°C and continuously gassed with oxygen (medical quality).

After the collagenase perfusion, the liver tissue was gently teared apart and cells were dispersed by shaking. The loosened mass and cell suspension were then passed through a 250 µm nylon filter. The filtrate was cooled on ice and centrifuged at 50xg for 5 minutes at 4°C. The cell pellets were resuspended in Williams E medium (Gibco, Betehesda, MD, USA) containing 10 % fetal calf serum (Gibco). The suspension was passed through a 100 µm nylon filter and the viability of the cells was determined by Trypan Blue exclusion test (Gibco).

### Cell culture:

Cells were seeded in 75 cm² tissue culture flasks, coated with human liver extracellular biomatrix (ECM) at a density of 150.000-175.000 viable cells/cm². Cells were cultured at 37°C in a humid atmosphere with 5% CO₂. Between 12-18 hours after cell seeding, the flasks were washed with PBS to remove dead or unattached cells and the medium refreshed. During the whole culture procedure, the medium was refreshed three times a week.

Human hepatocytes and the developed hepatocyte cell lines were cultured in Williams's Medium E (Gibco) supplemented with:
- 10 % Fetal calf serum (South American origin, Gibco)
- 2 mM L-glutamine (Gibco)
- 20 mU/ml Insulin (Actrapid HM: Novo Nordisk Pharma)
- 50 nM Dexamethasone Phosphate (Decadron: Merck Sharp & Dhome)
- 2.5 µg/ml Fungizone (Fungizone Ad Perfusionem: Bristol-Myers Squibb.)
- 50 µg/ml Gentamycin (Geomycine Injectable: Shering-Plough)
- 100 µg/ml Vancomycin (Vancocin 500: Lilly)
- 100 U/ml Penicillin (BenzylPenicillinum natricum: Continental Pharma)
- 100 µg/ml Streptomycin (Streptomycinsulphate: Heyl Chem. Pharma. Berlin).

In the first weeks of cell culture a near confluent monolayer of hepatocytes could be observed by phase-contrast microscopy. After several weeks in culture (3-15) colonies of small hepatocyte-like cells could be observed in some flasks. When such colonies grew to larger cell clusters, they were passaged to a 24-well tissue culture plate, precoated with ECM.

Cells were detached from the tissue culture flask by incubation with Trypsine-EDTA (5 min, 37°C) (Gibco), the cell suspension was diluted with culture medium and centrifuged (50xg, 5 min, 4°C). The cell pellet was resuspended in culture medium and the number of cells determined.

Growing cells were passaged several times. During these passages the culture medium was monitored for the production of human serum albumin (HSA) by a human-specific ELISA technique. Cultures which did not produce HSA were discarded. Cultures with high production of HSA were subcloned to obtain single-cell colonies.

To obtain single-cell colonies cells were seeded in 96-well plates, coated with ECM at the following cell densities: 10 cells/well, 3 cells/well, 1 cell/well and 0.3 cells/well (48 wells each). If in a certain well a single cell stood at the basis for the development of a colony and these cells produced HSA, that cell line was further developed.

The cell lines were further characterized for a number of hepatocyte specific features:
a) Cytokeratin expression pattern;
b) Cellular enzyme activity;
c) Plasma protein synthesis and influence of acute phase response by IL-6;
d) Bilirubin conjugation;
e) Cytochrome P₄₅₀ enzyme activity.

During the process of developing and after the establishment of human hepatocyte cell lines, cells were cryopreserved using a modification of the technique as described previously for adult human hepatocytes (Rijntjes et al., supra).

### Extracellular biomatrix:

Extracellular biomatrix (ECM) of normal human liver was prepared according to the methods described by Roykind et al. (J Cell Biol 87, 255-263, 1980) and Moshage et al (supra). After isolation, the extracellular matrix was lyophilised and dissolved in 0.1 M acetic acid in a final concentration of 200 µg protein per ml. This suspension was layered on tissue culture plastic (20 µg/cm²) and the acetic acid was allowed to evaporate. The culture plastic, precoated with ECM, was washed with PBS prior to use.

### Deep freezing storage of hepatocyte cell lines:

Trypsine-EDTA was used to detach the cells from the culture flask. Cells were centrifuged (50xg, 5 min, 4°C). The cells were suspended in ice-cold culture medium containing 10% (v/v) dimethylsulfoxide (DMSO) and 20% (v/v) fetal calf serum in a density of 5-10 x 10⁶ cells/ml. This suspension was placed at -80°C for 4-6 hours after which the vials were stored in liquid nitrogen vapour phase (-196°C) for long-term storage. At regular intervals cells were thawed and cultured in order to determine the influence of cryopreservation on the viability and the characteristics of the hepatocyte cells.

Hepatocytes were thawed by immersion of a storage vial in a 37°C water bath. Immediately after thawing the cells were rapidly diluted with culture medium at 4°C and washed to remove the cryoprotectant by centrifugation at 50xg for 5 min at 4°C. In general, one vial (5-10 x 10⁶ cells) was seeded in one 75 cm² tissue culture flask.

### Evaluation of the tumorigenicity:

The tumorigenicity of the hepatocyte cell lines was tested in severe combined immunodeficiency (SCID) mice, which have small lymphpoid organs and are deficient for B and T lymphocyte functions. The mice were bred in microisolator cages under specific pathogen-free conditions at a temperature of 23°C and air humidity of 40-70% and were given irradiated food and autoclaved water ad libitum. The cage bedding material was autoclaved. Aseptic procedures were routinely performed under a laminar flow hood and animals were treated following the institution's guide for the care and use of laboratory animals.

Ten million cells of the immortalised cell lines were injected subcutaneously into four-week-old female SCID mice. For each of the cell lines, 5 mice were studied. After injection, mice were observed weekly until 6 months for the presence of tumour formation at the site of injection, unless their general condition deteriorated. Before and at the end of follow-up the weight of the SCID mice was recorded. Mice were sacrificed after 6 months by cervical dislocation. Necropsy was performed with inspection of the lungs, liver, kidneys, spleen, intestines and retroperitoneal space. The results of tumour formation in de different groups was analysed by the Mann-Whitney test and a p-value < 0.05 (two-tailed) was considered significant. Tumour tissue was examined by standard histopathological techniques employing haematoxylin-eosin (HE) staining on paraffin-embedded tumour sections.

### Immunocytochemical detection of cytokeratin:

Confluent cell monolayers of the immortalized cell lines, cultured on LabTek culture chambers (Miles Laboratories INC, Napeville, Illinois, USA and purchased from Gibco BRL, Bethesda MD, USA) were air-dried and subsequently fixed in acetone (10 minutes at room temperature), air-dried again and stored at -20°C until use. A three-step, indirect immunoperoxydase procedure (5) was performed for cytokeratin staining of these cells using a various monoclonal antibody against CK7 (dilution 1:5), CK8 (1:10), CK18 (1:5) and CK19 (1:10) all purchased from Amersham (Amersham, UK).

### Human Serum Albumin (HSA) determination:

A specific monoclonal antibody (13E3) (developed at the laboratory of the inventor) directed against HSA (no cross-reactivity was observed with mouse or bovine serum albumin) was adsorbed onto 96-well microtiterplates (0.2µg/well) (Microlon ELISA plates, Greiner) for 16-18 hours at 4°C. After blocking the non-specific binding sites of the wells with 1% Blotto in PBS (1 hour at room teperature), serum samples were diluted according to their HSA content and incubated (100 µl/well, 1 hour at room temperature). Bound HSA was detected by addition of a rabbit polyclonal antibody, directed against HSA and conjugated with horseradish peroxidase (Dako,, 1:5000, 100 µl/well, 30 min at room temperature). Finally, colour development was performed using tetramethylbenzidine (Boehringer, Mannheim, Germany) and stopped using 1M H₂SO₄. After each incubation the plate was washed with PBS-Tween 20 (0.05%) and all dilutions were made in PBS containing 0.1% Blotto. On each test plate negative controls and standard series were included.

### Human fibrinogen determination:

A specific monoclonal antibody directed against human fibrinogen (clone FG21, Sigma, St. Louis, MO, USA) was adsorbed onto 96-well microtiterplates (0.2µg/well) (Microlon ELISA plates, Greiner) for 16-18 hours at 4°C. After blocking the non-specific binding sites of the wells with 1% Blotto in PBS (1 hour at room teperature), serum samples were diluted according to their fibrinogen content and incubated (100 µl/well, 1 hour at room temperature). Bound human fibrinogen was detected by addition of a rabbit polyclonal antibody, directed against fibrinogen and conjugated with horseradish peroxidase (Dako,, 1:5000, 100 µl/well, 30 min at room temperature). Finally, colour development was performed using tetramethylbenzidine (Boehringer, Mannheim, Germany) and stopped using 1M H₂SO₄. After each incubation the plate was washed with PBS-Tween 20 (0.05%) and all dilutions were made in PBS containing 0.1% Blotto. On each test plate negative controls and a standard series were included.

### Cytochrome P₄₅₀ enzyme activity:

Cytochrome P₄₅₀ enzyme activity was determined by measuring the disappearance of lidocaine from the culture medium together with the formation of the metabolite monoethylglycinexylidide (MEGX). Lidocaine (Sigma) was added to culture medium at a concentration of 20 µg/ml and after 24 hours medium samples were collected. Cells were also harvested for DNA determination. For the determinations of lidocaine and MEGX commercial assays were used (Lidocaine and MEGX kits for determination on TDx, Abbot laboratories).

### Cell lysis and DNA measurement:

Cell pellets, obtained after scraping of cell cultures, were lysed by ultrasonification. After lysis the remaining cell debris was removed by centrifugation. The DNA content of the cells was measured by the DAPI method (Kapuseinski et al., Anal Biochem 83, 252-257, 1977).

### Routine enzyme determinations:

Serum enzymes (transaminases (AST and ALT), lactate dehydrogenase (LDH), ammonia and γ-glutamyl transpeptidase (γ-GT)) were determined on a clinical laboratory analyser.

### Bilirubin analysis:

Cells were cultured in William's E medium supplemented with 35 µM unconjugated bilirubin (UCB) (Sigma). Unconjugated bilirubin was stabilized with bovine serum albumin (BSA) (Sigma) in a molar ratio of UCB : BSA = 1 : 2. At different time points samples were drawn from the cultures and analyzed for the formation of mono- and di-esterconjugates of bilirubin by liquid chromatochraphy (Muraca and Blankaert, Clin Chem 29, 1767-1771, 1983). After 24 hours, cultures were washed and the cellular DNA was determined as described above.

### 2. Results

### Isolation of human hepatocyte cell lines

In the first weeks of cell culture a near confluent monolayer of hepatocytes could be observed by phase-contrast microscopy. After several weeks in culture (3-15) colonies of small hepatocyte-like cells could be observed in some flasks. When such colonies grew to larger cell clusters, they were passaged to a 24-well tissue culture plate, precoated with ECM using trypsine-EDTA (Gibco). Growing cells were passaged several times and during these passages the culture medium was monitored for the production of human serum albumin (HSA) by a human-specific ELISA technique. Cultures which did not produce HSA were discarded. Cultures with high production of HSA were subcloned to obtain single-cell colonies. By this method, a number of cell lines have been established from livers of different donors.

From only the minority of the normal human livers, of which cells were isolated, cell lines could be developed. During the first year after isolation, in the majority of cell clusters or in isolated cell lines the cell division halted, the cells greatly enlarged, the number of their nucleoli increased and eventually the cells died. Only from a limited number of the initially observed cell clusters, immortalized cell lines could be developed.

Cells were cultured on ECM during the first passages. However, after the establishment of single-cell lines some of the cell lines could also be adapted to growth in culture flasks (Nunc: Gibco) without coating with ECM. The absence of ECM appeared to have no influence on the cell parameters investigated.

The characteristics of the cells after different numbers of passages have been determined. After one year in culture, most of the cell lines appeared stable and homogenous by phase-contrast microscopic observation. In addition, cytokeratin expression patterns and HSA synthesis levels did not change with the number of cell passages.

### Tumorigenicity of cell lines:

12 cell lines have been tested for tumor formation in SCID mice. For each cell line at least 5 SCID mice were injected subcutaneously with 10x10⁶ viable cells. The original cell lines B and C produced tumors in SCID mice. Of the 10 subclones tested so far, 2 did not produce tumors (table 1), which were further developed. Onset of tumor formation in the mice injected with the other 8 cell lines was between 1 and 4 months after injection.

**Table 1**

| Cell line | time to onset tumor formation | number of mice with tumors |
|---|---|---|
| B | > 6 months | 1/5 |
| BD5 (invention) | > 4 months* | 0/5 |
| C | 1-4 months | 5/5 |
| EA4 (invention) | > 7 months | 0/6 |

| | | |
|---|---|---|
| * so far investigated. | | |

### Characterization of cell lines in comparison with HepG2 cells and primary human hepatocytes:

### a) Cytokeratin (CK) expression:

The pattern of CK expression revealed that the cell lines A, B, C and E combined a low level of CK7 with a high level of CK8 and CK18 expression which is a characteristic pattern for adult human hepatocytes. The cell line D showed a high positivity for CK7, which is normally associated with bile duct epithelial cells.

The immortalized hepatocyte cell line thus showed a normal pattern of CK expression when compared to primary human hepatocytes.

**Table 2**

| cells | CK-7 | CK-8 | CK-18 | CK-19 |
|---|---|---|---|---|
| prim. human hepatocytes | neg. | 60% | 95% | neg. |
| HepG2 | neg. | 10% | 90% | < 1% |
| A (invention) | < 1% | 95% | 100% | 90% |
| B and subclones (invention) | < 1% | 95% | 100% | 40% |
| C and subclones (invention) | < 1% | 50% | 100% | 95% |
| D | 80% | 80% | 100% | 100% |
| E and subclones (invention) | < 1% | 100% | 100% | n.d. |
| n.d. : not determined | | | | |

### b) Cellular enzyme activity:

Measurement of enzyme activity in the supernatant of cell lysate indicated that all cell lines showed a high level (3 to 6-fold) of γ-GT expression and a low ALT and AST expression, as compared to freshly prepared human hepatocytes. The AST/ALT ratio remained, however, relatively constant. The expression level of LDH was practically the same for the cell lines when compared to freshly prepared human hepatocytes (table 3).

**Table 3**

| cells | ALT | AST | γ-GT | LDH | AST/LDH |
|---|---|---|---|---|---|
| prim. hepat. | 0.86 | 13.59 | 0.33 | 5.96 | 15.8 |
| Hepg2 | 0.05 | 1.14 | 1.35 | 7.43 | 22.8 |
| A | 0.14 | 1.67 | 1.03 | 3.08 | 11.9 |
| B | 0.12 | 2.78 | 1.83 | 7.88 | 23.2 |
| C | 0.10 | 1.31 | 1.31 | 9.44 | 13.1 |
| D | 0.14 | 2.44 | 1.37 | 7.16 | 17.4 |

### c) HSA secretion and induction of acute phase response by IL-6 on fibrinogen.

**Table 4**

| Protein secretion as expressed in ng/µg DNA/24 h | | | |
|---|---|---|---|
| Cells | HSA | fibrinogen - IL-6 | fibrinogen + IL-6 |
| prim. hepat. | 120 | 310 | n.d. |
| HepG2 | 60 | 6.8 | 68 |
| A | 70 | 0.7 | 30 |
| B | 270 | 5.1 | 205 |
| C | 70 | 4.1 | 41 |
| D | 170 | 2.4 | 108 |
| n.d. + not determined | | | |

HSA and fibrinogen production was measured in cultures 24 hours after refreshing the culture medium. The HSA production level of the cell lines B and D was significantly higher than of HepG2 cells. The fibrinogen production, which is low in these cell lines, could be strongly enhanced by IL-6 treatment, a known inducer of an acute phase response in hepatocytes. After a three day stimulation with 2000 U/ml rIL-6, the fibrinogen production rose to about 10-40-fold of the initial production, reaching levels which were comparable with the amount primary human hepatocytes produced shortly after isolation. HSA levels dropped to about 40-50% after IL-6 treatment.

### d) Bilirubin conjugation

**Table 5**

| Conjugation of bilirubin in µmol/µg DNA/24 h | | | | |
|---|---|---|---|---|
| cells | total | mono-conjugate | di-conjugate | ratio di-/mono- |
| prim. hepat. | 0.79 | 0.55 | 0.24 | 0.44 |
| HepG2 | 0.45 | 0.31 | 0.14 | 0.42 |
| A | 0.25 | 0.17 | 0.09 | 0.53 |
| B | 1.38 | 0.77 | 0.61 | 0.79 |
| C | 0.47 | 0.23 | 0.24 | 1.04 |
| D | 1.15 | 0.56 | 0.59 | 1.05 |

As shown in table 5, the cell lines B and D were able to conjugate a higher amount of bilirubin in 24 hours than primary human hepatocytes. Moreover, they conjugated substantially more bilirubin to its di-ester conjugate.

### e) Cytochrome P₄₅₀ enzyme activity

Cytochrome P₄₅₀ activity was assayed by measuring the conversion of lidocaine to MEGX. Therefore, lidocaine was added to fresh medium at a concentration of 20 µg /ml. Lidocaine and MEGX concentrations were measured after 24 hours. Table 6 shows that the cell lines A and B removed lidocaine at a substanial higher rate (3-fold) than HepG2 cells; however, this was still about 30% of those found in primary cultures of human hepatocytes. The production of MEGX, however, was for all cell lines considerable lower (only 2-3%) when compared to human hepatocytes. This may be due to the conversion of lidocaine to other methabolites such as glycinexylidide or hydroxylated forms of lidocaine for which we had no assay available.

**Table 6**

| Lidocaine removal and MEGX production (ng/µg DNA/24 h) | | |
|---|---|---|
| cells | lidocaine | MEGX production |
| prim. hepat. | 1080 | 305 |
| HepG2 | 100 | 6 |
| A | 310 | 10 |
| B | 310 | 9 |
| C | 90 | 5 |
| D | 210 | 7 |

Furthermore, cytochrome P₄₅₀ enzyme activity was evaluated using a number of isoenzyme specific substrates (table 7)

**Table 7**

| | |
|---|---|
| Substrate | CYP P-450 |
| ethoxycoumarine | general |
| coumarine | 2A6 |
| testosterone | 3A4 |
| tolbutamide | 2C8, 9, 10 |
| dextromorphan | 2D6 |
| chlorohexazone | 2E1 |
| lubeluzole | 2D6, 3A4 |

Cell lines were screened using for testosterone metabolism (3A4), dextromethorphan O-demethylation (2D6) and 7-ethoxycoumarine hydroxylation (general CYP). These substates were chosen because of the importance of the corresponding CYP-isoenzymes in drug metabolism. In addition, two experimental drugs of which the CYP-isoenzymes responsible for metabolsm are known, were investigated. R051619 (3A4, 2A6) and R110231 (3A4, 2D6, 2C9 and 2C19) (table 8).

The metabolism of in number of substrates was investigated in the different cell lines and compared with primary human hepatocytes and the human hepatoblastoma cell line HepG2. The cell lines of the invention (B, C and E) all showed a higher rate of metabolism for the different substrates than the widely used HepG2 cells (2-15 times). A comparison was made between primary human hepatocytes and the cell line BD5 (table 9).

**Table 8**

| Cell lines | Substrates | | | | | | |
|---|---|---|---|---|---|---|---|
| | **Testosterone** | **7-Ethoxy-coumarine** | **R051619** | | **R110231** | **Dextromethorphan** | **Dextromethorphan** |
| | Overall metabolism % | % | Overall metabolism % | Nor-R051619 % | Overall metabolism % | Overall metabolism % | Dextrorphan % |
| Hum.Hept | 93.4 | 1.87. 0.42. 0.43 | 36.2 | 13.5 | 81.9 | 29.8, 69.2, 26.5 | 14.2, 11.9, 13.9 |
| Hep G2 | 47.7 | 0.009 | - | - | 40.2 | - | - |
| B | 96.4 ± 3.8 | 0.111 ± 0.043 | 8.7 ± 7.5 | 2.4 ± 1.2 | 84.1 ± 16.9 | 9.7 | 6.4 |
| C | 96.3 ± 4.5 | 0.149 ± 0.027 | 3.4 ± 2.4 | 1.5 ± 0.4 | 54.8 ± 5.4 | - | - |
| E | 90.7 ± 3.8 | 0.059 ± 0.005 | 15.4 ± 3.2 | 4.4 ± 1.1 | 97.0 ± 2.5 | 10.5 ± 2 | 2.5 ± 1.1 |

**Table 9**

| | | | **Hum. Hept.** | **Sub-Clone BD5** |
|---|---|---|---|---|
| **Compound** | Metabolite | CYP involved | % Overall metabolism / % Metabolite | % Overall metabolism / % metabolite |
| | | | | |
| **Testosterone** | | 3A4 | 93.4 | 82.1 |
| | Metabolite 1 | 3A4 | 2.5 | 5.1 |
| | Metabolite 2 | 3A4 | 1.5 | 1.2 |
| | Metabolite 3 | 3A4 | 11.5 | 3.0 |
| | Metabolite 5 | ? | 78.0 | 72.8 |
| **RO51619** | | 3A4 | 36.2 | 16.4 |
| | Nor-R051619 | 3A4 | 13.5 | 3.7 |
| **R110231** | | 3A4, 2D6, 2C9, | 81.9 | 51.8 |
| | | 2C19 | | |
| **7-ethoxycoumarine** | | general | 1.87, 0.42, 0.43 | 0,191, 0.032 |
| **Dextromethorphan** | | 2D6 | 29.8, 69.2, 26.5 | 9.7 |
| | Dextrorphan | 2D6 | 14.2, 11.9, 13.9 | 6.4 |
| **Lubeluzole** | | | - | 61.8 |
| | glucuronide | | - | 40.4 |
| | 6-hydroxymetabolite | 2D6 | - | 5.5 |
| | normetatolite | 3A4 | - | 1.8 |
| **Chlorzoxazone** | | 2E1 | | 6.6 |

The presence of CYP 3A4 and CYP 2D6 could be further confirmed by the addition of specific inhibitors (Ketoconazole: CYP3A4 and Quinidine: CYP 2D6). Cytochrome c reductase activity, required for the functioning of cytochrome P450 enzymes, could be demonstrated in all of the cell lines at levels 10.9 - 67.7 nmol cytochrome c /min/mg protein.

### 3. Discussion

Starting from primary liver cells, a number of immortalized human hepatocyte cell lines were developed by long-term culture and careful selection. These cell lines were characterized for cytokeratin expression patterns (table 2), cellular enzyme activity (table 2), the ability to produce human serum albumin and fibrinogen, as well as the response to IL-6 (table 4). In addition bilirubin conjugation (table 5) and cytochrome P₄₅₀ enzyme activity (table 6-9) were studied.
From these studies it is concluded that unique immortalized human hepatocyte cell lines with characteristics similar as those found in primary human hepatocytes have been developed. An important function, which hepatocytes normally loose after isolation is their cytochrome P₄₅₀ enzyme activity. In the present invention cell lines are provided which have conserved these function at 30-50% of the level seen in human hepatocytes which is considerably better than found in the HepG2 cells. In addition, many other hepatocyte functions have also been preserved (tables 2-5) and the cell lines were non-tumorigenic when injected into SCID mice (table 1), making them very suitable for application in a bioartificial liver.

### EXAMPLE 2

### A bioartificial liver based on immortalized human hepatocytes of the invention

The used bioartificial liver support system consists of a blood circuit with continuous plasma separation and a bioreactor circuit as described in WO 97/12960. The bioreactor comprises a solid support for use in cell cultivation in vitro, comprising a 3D matrix and hollow fibres being permeable to at least gaseous oxygen and/or gaseous carbon dioxide. The bioreactor is perfused with plasma, such that there is direct contact between the plasma and the hepatocytes with low diffusion gradients, thereby more closely resembling the in vivo situation. Because of the plasmapheresis patient immune competent cells do not come in contact with the hepatocytes.

Normally, primary hepatocytes do not proliferate in vitro. Nevertheless, according to the invention albumin secreting colonies were found in long-term cultures. From these colonies several cell lines were developed. These cell lines were investigated for the ability to synthesize albumin and fibrinogen and for bilirubin conjugation, cytochrome P-450 enzyme activity, presence of ALT, AST, γGT, LDH and cytokeratin expression. When compared to hepatoblastoma cells, these cell lines are superior in several or almost all of the aforementioned characteristics. The cells were also tested for tumorigenicity in SCID mice and proved to be non-tumorigenic.

The cells of the cell line according to the invention were seeded in the bioreactor and kept in culture till the total amount of hepatocytes was estimated to be about 5 billion cells.

The used model of ALF is based on total but reversible ischemia of the liver and is a modification of the model of graded ischemia as described by de Groot et al.(J Surg Res 42, 92-100, 1987).

The animals were female stress-negative Belgian landrace pigs weighing 18 to 22 kg. Surgery was performed under general anesthesia. After surgery and discontinuation of anesthesia animals were installed in a cage equipped with a three channel swivel-tethering system, allowing comfortable maintenance of fully ambulatory pigs although continuous blood pressure monitoring and infusion therapy. Animals were allowed to drink ad libitum.

### Preparative surgery:

On day zero a single lumen catheter was placed into the carotid artery for continuous blood pressure monitoring and blood sampling. A double lumen catheter was inserted into the external jugular vein for infusion of intravenous fluids. The catheters were tunneled and brought up to the dorsal neck.

On day three a xyphopubic laparotomy was performed. The liver was skeletonized excising its ligaments. All structures in the hepatoduodenal ligament were divided except the portal vein, the hepatic artery and the common bile duct. The common bile duct and the common hepatic artery, together with the inbranching gastroduodenal artery, were isolated and a vessel loop was put around the proper hepatic artery just at the bifurcation into the right and left liver branch and around the common bile duct. The right gastric artery was also surrounded by a vessel loop to prevent backflow. In some animals the gastroduodenal artery was also surrounded. These vessel loops were not tightened and were exteriorized through the abdominal wall. A side-to-side portocaval shunt was performed, followed by ligation of the portal vein close to the liver hilum to create a functional end-to-side shunt.

### Induction of ischemia:

After recovery, 72 hours postoperatively, the vessel loops around the common bile duct, the hepatic artery, the right gastric artery and, in case, around the gastroduodenal artery were tightened without anesthesia or sedation. After 10 hours the loops were cut and pulled off.

After the induction of ischemia, behaviour, as well as respiration rate, pulse and blood pressure was controlled every hour. Blood samples for blood sugar, ionogram, blood ureum, blood ammonia, serum lactate, serum alkaline phosphatase, serum GOT, serum GPT, serum LDH, albumin, clotting factors, blood gases and blood count were taken regularly. A postmortem was done as soon as the animals died.

### Animal treatment protocol:

The animals were divided in three groups. Group I pigs were not connected to any extracorporeal system. Group II pigs were connected to a complete BAL system without hepatocytes. Group III pigs were treated with a BAL loaded with on average 5 billion of hepatocytes. These therapies were initiated 9 hours after induction of ischemia. Plasmapheresis was performed for maximally 72 hours or shorter if death occured earlier. When treated with a loaded BAL system, the BAL was renewed after initial treatment.

### Results:

Prolongation of life served as a primary endpoint of BAL therapy. All animals of group I died between 12 and 17 hours after induction of ischemia. In all pigs death was due to hepatic coma. Animals of group II died between 12 and 24 hours after induction of ischemia. With 24 hours of BAL treatment survival time raised up to 33 hours. When BAL treatment with a single bioreactor was continued till death, survival time reached 46 hours. When the bioreactor was renewed after 12 hours and the treatment was continued for another 60 hours, the pigs survived with complete normalisation of behaviour and biochemical parameters. The pigs were sacrificed at day 7. On histology the liver showed typical changes of regeneration.

From these results it can be concluded that the cell lines of human immortalized hepatocytes of the invention can be used in a bioartificial liver system for the treatment of acute liver failure, by significantly prolonging survival time accompanied by an important improvement of biochemical parameters and of neurological status.

## Claims

1. Cell line of human immortalized hepatocytes for medical use.

2. Cell line of human immortalized hepatocytes for the treatment of liver failure.

3. Cell line of human immortalized hepatocytes for use in a bioartificial liver system for the treatment of liver failure.

4. Cell line according to claims 1-3 **characterized in that** the hepatocytes of the cell line are non-tumorigenic.

5. Cell line according to any of the preceding claims **characterized in that** the cytochrome P₄₅₀ enzyme activity of the hepatocytes of the cell line is at least 30%, preferably at least 50% of the level of primary human hepatocytes.

6. Cell line according to any of the preceding claims **characterized in that** the cell line is obtainable by the process as described in Example 1.

7. Cell line according to any of the preceding claims **characterized in that** the cell line is the HHY-B cell line deposited on March 23, 1999, at the Belgian Coordinated Collections of Microorganisms and having the deposit accesion no LMBP 4981 CB.

8. Cell line according to claims 3-7 **characterized in that** the bioartificial liver system comprises a solid support for use in cell cultivation in vitro, comprising a 3D matrix and hollow fibres being permeable to at least gaseous oxygen and/or gaseous carbon dioxide.

9. Cell line according to claim 2-8 **characterized in that** the liver failure is acute liver failure.

10. Cell line according to claim 2-8 **characterized in that** the liver failure is chronic liver failure.

11. Use of a cell line of immortalized human hepatocytes for the preparation of a bioartificial liver system for the treatment of liver failure.

12. Use according to claim 11 **characterized in that** the hepatocytes of the cell line are non-tumorigenic.

13. Use according to claim 11 or 12 **characterized in that** the cytochrome P₄₅₀ enzyme activity of the hepatocytes is at least 30%, preferably at least 50% of the level of primary human hepatocytes.

14. Use according to claim 11, 12 or 13 **characterized in that** the cell line is obtainable by a process as described in Example 1.

15. Use according to claims 11-14 **characterized in that** the cell line is the HHY-B cell line deposited on March 23, 1999, at the Belgian Coordinated Collections of Microorganisms and having the deposit accesion no LMBP 4981 CB.

16. Use according to claims 11-15 **characterized in that** the bioartificial liver system comprises a solid support for use in cell cultivation in vitro, comprising a 3D matrix and hollow fibres being permeable to at least gaseous oxygen and/or gaseous carbon dioxide.

17. Use according to claims 11-16 **characterized in that** the liver failure is acute liver failure.

18. Use according to claims 11-16 **characterized in that** the liver failure is chronic liver failure.

19. Bioartificial liver system comprising a solid support for use in cell cultivation in vitro, comprising a 3D matrix material and hollow fibres being permeable to at least gaseous oxygen and/or gaseous carbon dioxide, provided with a cell line of human immortalized hepatocytes.

20. Bioartificial liver system according to claim 19 **characterized in that** the hepatocytes of the cell line are non-tumorigenic.

21. Bioartificial liver system according to claim 19 or 20 **characterized in that** the cytochrome P₄₅₀ enzyme activity of the hepatocytes of the cell line is at least 30%, preferably at least 50% of the level of primary human hepatocytes.

22. Bioartificial liver system according to claims 19, 20 or 21 **characterized in that** the cell line is obtainable by the process as described in Example 1.

23. Bioartificial liver system according to claims 19-22 **characterized in that** the cell line is the HHY-B cell line deposited on March 23, 1999, at the Belgian Coordinated Collections of Microorganisms and having the deposit accesion no LMBP 4981 CB.

24. Bioartificial liver system according to claims 19-23 for use in the treatment of liver failure.

25. Bioartificial liver system according to claim 24 **characterized in that** the liver failure is acute liver failure.

26. Bioartificial liver system according to claim 24 **characterized in that** the liver failure is chronic liver failure.

27. Bioartificial liver system according to claims 24-26, wherein the treatment consists of the extracorporal superfusion of the hepatocytes of the cell line, which are provided in the bioartificial system, by body fluid of a patient, and subsequently returning the body fluid to the patient.

28. A permanent cell line of human hepatocytes for use in replacing and/or supporting the in vivo liver function for increasing survival of animal subjects with a liver disorder **characterized in that** the cells are immortalized without viral transfection or transformation and are non-tumorigenic.
